(19) Europäisches Patentamt
European Patent Office
Office européen des brevets

(11) **EP 1 293 184 B1**

(12) **EUROPEAN PATENT SPECIFICATION**

(45) Date of publication and mention
of the grant of the patent:
**27.07.2005 Bulletin 2005/30**

(51) Int Cl.⁷: **A61F 9/08**, A61H 3/06

(21) Application number: **02019374.4**

(22) Date of filing: **29.08.2002**

(54) **Walking auxiliary for person with dysopia**

Gehhilfsmittel für sehbehinderte Personen

Moyen auxiliaire de marche pour des personnes souffrant de déficiences visuelles

(84) Designated Contracting States:
**FR GB**

(30) Priority: **17.09.2001 JP 2001281519**

(43) Date of publication of application:
**19.03.2003 Bulletin 2003/12**

(73) Proprietor: **SEIKO EPSON CORPORATION**
**Tokyo 160-0811 (JP)**

(72) Inventor: **Sato, Shigemi**
**Suwa-shi, Nagano-ken 392-8502 (JP)**

(74) Representative: **Hoffmann, Eckart, Dipl.-Ing.**
**Patentanwalt,**
**Bahnhofstrasse 103**
**82166 Gräfelfing (DE)**

(56) References cited:
**US-A- 5 636 038**          **US-B1- 6 198 395**

**Description**

This invention relates to a walking auxiliary for a person with dysopia for detecting obstacles when the person takes a walk.

When a person with dysopia takes a walk, he uses a white stick to detect obstacles and avoid those obstacles.

There is a problem with the white stick described above in that the stick can only catch an object at one point, therefore it gives insufficient information and cannot ensure full safety. Moreover, there are problems in that when the user of the stick stands on a flat and broad road surface, he does not know where he may walk because there are no characteristic targets around him, and he also cannot recognize a distant scene, and so on.

A walking auxiliary according to the pre-characterizing portion of claim 1 is disclosed in US-A-5,636,038. This prior art, for converting the visual and/or auditory into tactile representations, includes imaging equipment for converting light and/or sounds, including spoken text, into electrical signals, processing equipment for processing the electrical signals, and a tactile display for converting processed electrical signals into tactile images. The tactile images are felt by the user enabling them to obtain visual or auditory information by touch about the world around them that would otherwise be obtained through vision and/or hearing. The processing means in essence identifies objects, their relative size, their spatial location relative to the device, their movement, if any, etc.

US-A-6,198,395 discloses a sensory system and method for a visually impaired user including an array of laser transmitters for transmitting laser signals in given areas corresponding to each of the laser transmitters. An array of laser sensors are provided for receiving laser signals reflected from objects in the corresponding given areas with each laser sensor corresponding to a respective one of the array of laser transmitters. The time between transmitted and received laser signals of a laser transmitter and the respective laser sensor is indicative of the distance between the user and an object in the corresponding given area. A processor is operable with the laser transmitters and laser sensors to effect scanning of the given areas by the array and to process the transmitted and received signals to determine the distance between the user and an object in the given areas. A feedback system is operable with the processor for generating an audible or tactile feedback signal to the user for each of the laser sensors as a function of the received laser signals. The sensing system and method may also include an array of ultrasonic transducers.

This invention seeks to provide a walking auxiliary for a person with dysopia which enables the person to obtain sufficient information about obstacles and so on when he takes a walk.

This object is achieved with a walking auxiliary as claimed in claim 1. Preferred embodiments of the invention are subject-matter of the dependent claims.

Fig. 1      A block diagram showing the circuit construction of a walking auxiliary.

Fig. 2      A diagram of an auxiliary incorporating the circuit of Fig. 1.

Fig. 3      An perspective drawing showing one actuator of the actuator array of Fig. 1.

Fig. 4      A circuit block diagram of the actuator control unit and the actuator array of Fig. 1.

Fig. 5      A flow chart showing the actions of the image processing unit of Fig. 1.

Fig. 6      A diagram showing the method for finding the distance to the picked up object in the image processing unit of Fig. 1.

Fig. 7      A schematic diagram showing an example of a bicycle ahead of the user.

Fig. 8      A diagram showing an example of a hole and a tree ahead of the user.

Fig. 9      A diagram showing an example of a ball flying at the user.

Fig. 10      A block diagram showing the circuit construction of an exemplary auxiliary.

Fig. 11      A block diagram showing the circuit construction of an exemplary auxiliary.

Fig. 12      A block diagram showing the circuit construction of an examplary auxillary.

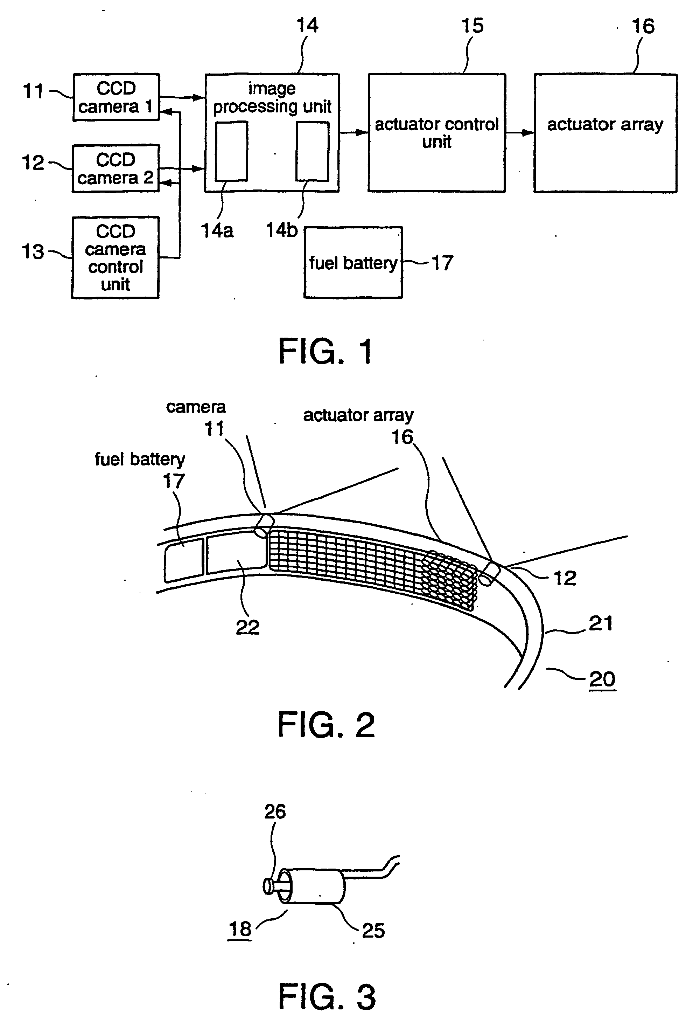

Fig. 1 is a block diagram showing the circuit construction of a walking auxiliary for a person with dysopia. The walking auxiliary for a person with dysopia (called "auxiliary" hereafter) comprises two CCD cameras 11, 12, a CCD camera control unit 13, an image processing unit 14, an actuator control unit 15, an actuator array 16, and a battery 17 as power source. The two CCD cameras 11, 12 are controlled by the CCD camera control unit 13, pick up images at different angles, respectively, and output their image pickup signals to the image processing unit 14. The image processing unit 14 is composed of a distance-measuring operation part 14a and a control signal forming operation part 14b. Although its details will be described later, the image processing unit 14 receives the image signals from the CCD cameras 11, 12 to perform image processing and measure the distance, forms the stereo image information (three-dimensional information), further converts the stereo information to two-dimensional information, forms a control signal for controlling the actuator array 16 and outputs it to the actuator control unit 15. The actuator control unit 15 drives the actuator array 16 and informs the user about the surrounding conditions picked up by the two CCD cameras 11, 12.

Fig. 2 is a perspective diagram of an auxiliary 20 incorporating the circuit of Fig. 1. This auxiliary 20 is provided with a headband 21, and the two CCD cameras 11, 12 are mounted to this headband 21 at a predetermined spacing. The actuator array 16 is mounted between the two CCD cameras 11, 12. The battery 17 is mounted to this headband 21, and a control unit 22 with built-in CCD camera control unit 13, image processing unit 14 and actuator control unit 15 is also mounted to this headband 21. This auxiliary 20 is used in a state in which the headband 21 is attached to the forehead of the user.

Fig. 3 is an perspective drawing with one activated actuator 18 of the actuator array 16. In the actuator 18, an exciting coil (not illustrated) is built in a cylinder 25 of about 1 mm in diameter, and a pin 26 supported movably in its axial direction between a protruding state and a retracted state is arranged in the cylinder 25. The pin 26 moves toward the forehead side of the user, when the actuator is activated by feeding an exciting current to the exciting coil of the cylinder 25, to transmit information to the user somatosensorially.

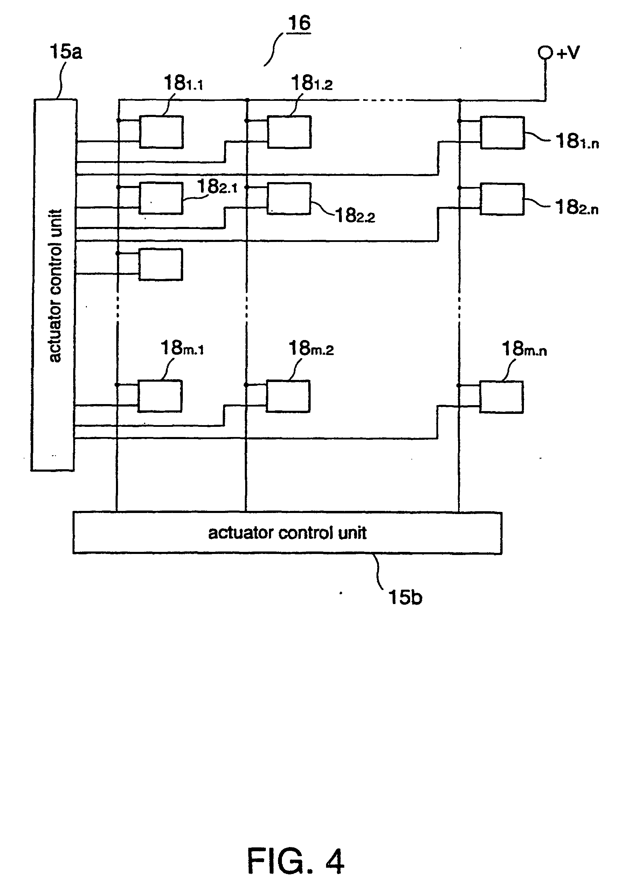

Fig. 4 is a circuit block diagram showing the relationship between the actuator control unit 15 and the actuator array 16. The actuator control unit 15 is composed of control units 15a and 15b. In the actuator array 16, actuators 18 ($18_{1.1}$, $18_{1.2}$ ..., $18_{1.n}$, $18_{2.1}$, $18_{2.2}$ ..., $18_{2.n}$, ... $18_{m.1}$, $18_{m.2}$ ..., $18_{m.n}$) are disposed in a matrix, the control unit 15a controls the row direction and the control unit 15b controls the column direction of this actuator array 16.

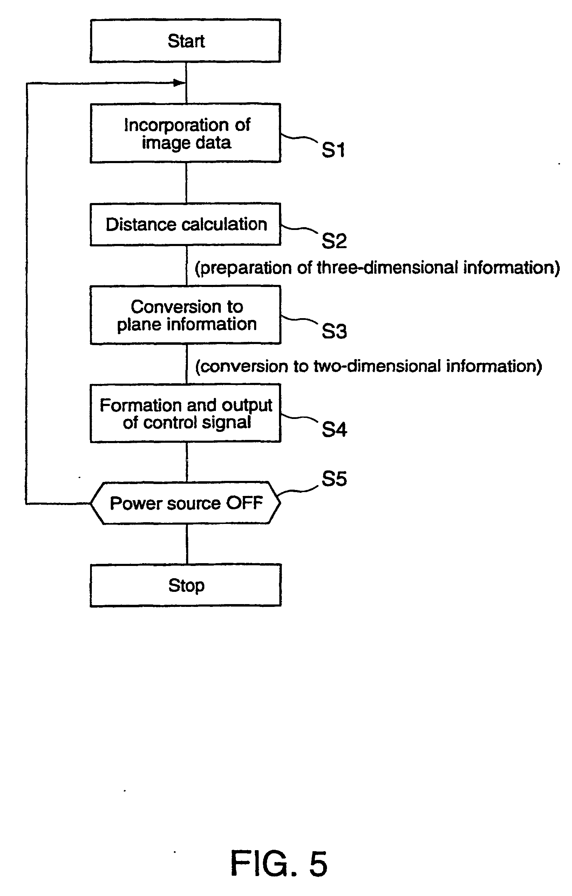

Fig. 5 is a flow chart showing the actions of the image processing unit 14.

(S1) The distance-measuring operation part 14a of the image processing unit 14 takes in image pickup signals which are picked up by the two CCD cameras 11, 12 at different angles, respectively.

(S2) The distance-measuring operation part 14a of the image processing unit 14 forms a three-dimensional image based on the image pickup signals. Therefore, first, it finds the distances from the user to the sites of a picked-up object based on the image pickup signals.

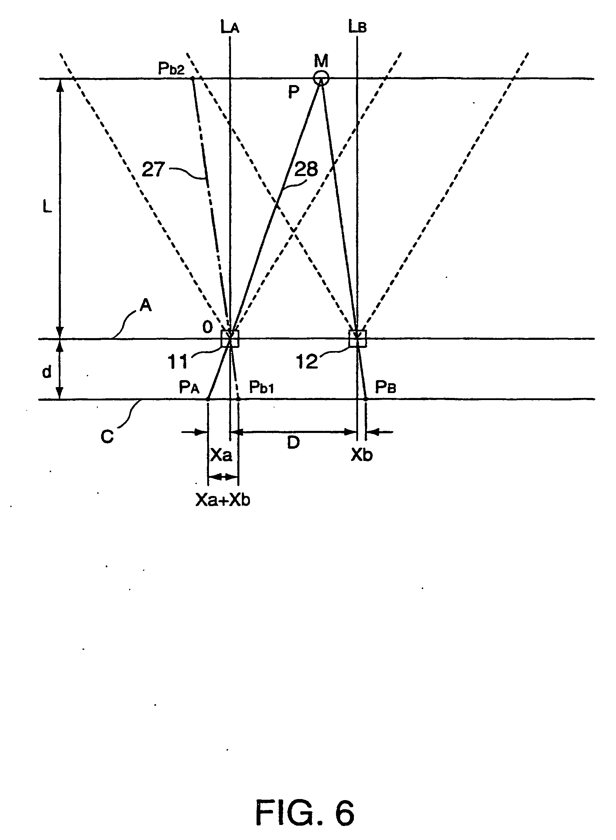

Fig. 6 is a diagram showing a method for finding the distance to a picked-up object. For example, some obstacle M is positioned at an illustrated point P. In this case, the position of point P comes into the field of view of both CCD cameras 11, 12. Accordingly, the CCD cameras 11, 12 project images of the obstacle M on respective imaging planes. In the CCD camera 11, an image of the obstacle M is formed on a point $P_A$ of an imaging plane C. Here, the deviation from the optical axis LA of this CCD camera 11 to the point $P_A$ is taken as $x_a$. In the CCD camera 12, an image of the obstacle M is formed on a point $P_B$ of the imaging plane C. Similarly to the CCD camera 11, the deviation between the optical axis LB of this CCD camera 12 to the point $P_B$ is taken as $x_b$. The distance-measuring operation part 14a of the image processing unit 14 calculates the above deviations $x_a$ and $x_b$, respectively.

Next, it is supposed that the optical axis of either one of the CCD cameras 11 and 12 is moved in parallel to make the optical axes LA and LB coincide with each other. Here, the optical axis LB of the CCD camera 12 is taken to be coincident with the optical axis LA of the CCD camera 11. If the optical axis LB is made coincident with the optical axis LA, a straight line connecting the obstacle M and the point $P_B$ of the imaging plane C is expressed by a double-dashed line 27 on the CCD camera 11 side. In this way, $\Delta OP_A P_{b1}$ and $\Delta OPP_{b2}$ can be formed between a straight line 28 connecting the obstacle M and the point $P_A$ of the imaging plane and the above double-dashed line 27 on the CCD camera 11 side. These $\Delta OP_A P_{b1}$ and $\Delta OPP_{b2}$ are similar triangles, therefore the following equation is established.

$$L/d = D/(X_a + x_b), \qquad (1)$$

so that

$$L = d \cdot D/(x_a + x_b). \qquad (2)$$

In the way described above, the distance-measuring operation part 14a of the image processing unit 14 obtains three-dimensional information thereby finding the distances for the picked up object in order. Moreover, the distance-measuring operation part 14a of the image processing unit 14 makes detection of the obstacle M (detection that the obstacle M (picked-up object) of image signal of the CCD camera 11 and the obstacle M (picked-up object) of image signal of the CCD camera 12 are the same object) and performs the above distance calculation. For example, if the head is slightly moved immediately after the power source is switched on, the visual field position of the distance-measuring operation part 14a changes, and the objects in the images obtained by the two CCD cameras 11 and 12 move in connection with the movement of head and the distance. It determines whether they are the same object by a calculation from this movement. Namely, it detects the obstacle M by use of the fact that the quantity of the position change of the left and right images to the movement of head always has a constant correlation if they are the same object (the calculation result takes an inherent correlation value) and the calculation result deviates from the correlation value if they are not the same object, when fixing the correlation of the two CCD cameras 11 and 12.

(S3) The control signal forming operation part 14b of the image processing unit 14 converts the above mentioned three-dimensional information to two-dimensional information. For example, a picked-up object located within a predetermined distance is extracted to give two-dimensional information of the picked-up object. At that time, the contour of the picked-up object is obtained to give two-dimensional information, when painting over the inside of the contour.

(S4) The control signal forming operation part 14b of the image processing unit 14 forms a control signal for controlling the actuator array 16 based on the above two-dimensional information. The actuator control unit 15 (15a, 15b) drives the actuator array 16 based on the control signal. For example, if the obstacle exists within a predetermined distance, an exciting current is fed to the actuator array 16 in a region equivalent to the two-dimensional shape of the obstacle. Pins 26 are pushed against the user's forehead to inform the user about the existence and shape of the obstacle. Since the actuators 18 are disposed in a matrix in the actuator array 16 as described above, the user can identify the shape of the obstacle by driving the actuators 18 in response to the plane shape of the obstacle.

(S5) The image processing unit 14 repeats the above processes (S1) to (S4) until the power source is turned off (or until a command of stop).

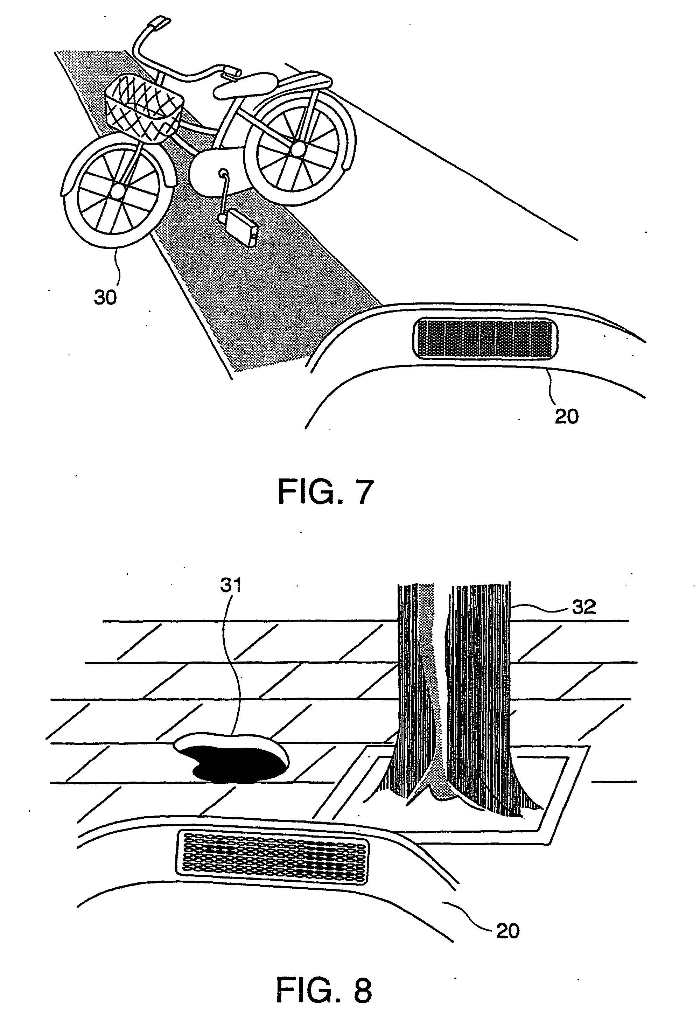

Fig. 7 is a schematic diagram showing a bicycle 30 ahead of the auxiliary. When the bicycle 30 is placed ahead, first, the distance is measured to obtain its three-dimensional information, then the three-dimensional information is converted to two-dimensional information, and the actuators of array 16 existing in a region corresponding to the two-dimensional information are driven to inform the user about the existence of the bicycle 30. Then, the region expands when the user walks toward the bicycle, therefore the user knows he is approaching the obstacle.

Above, an example wherein the control signal forming operation part 14b of the image processing unit 14 finds the contour of a picked-up object and gives this two-dimensional information in a state of painting over or filling the inside of the contour was illustrated, however, for example, when a dent or recession having a given size appears in a flat region (a state in which the distance only in a given area becomes far), it determines the dent as a hole and forms a control signal different from the above obstacle. For example, it forms and outputs a control signal for vibrating the corresponding part of the actuator array 16 at a predetermined period. The actuator control unit 15 (15a, 15b) drives the actuator array 16 and vibrates the pins 26 based on the control signal.

Fig. 8 is a drawing showing an example of a case where a hole 31 and a tree 32 exist ahead. The image processing unit 14 detects the hole 31 and forms a control signal for vibrating the actuator array 16 in a region corresponding to the hole, and the actuator control unit 15 (15a, 15b) drives and vibrates the actuator array 16 based on the control signal. For the tree 32, as illustrated above, the image processing unit 14 forms a control signal for vibrating the actuator array 16 in a region corresponding to the tree, and the actuator control unit 15 (15a, 15b) protrudes the pins 26 of the actuator array 16 based on the control signal.

In the above example, for instance, when the tree gets even closer, the image processing unit 14 forms a control signal different (e.g., in amplitude and/or frequency) from that in a more distant state to inform the user about an emergency and actuates the actuator array 16 not as usual to inform the user about an emergency.

In finding the contour of a picked-up object, the control signal forming operation part 14b of the image processing unit 14 stores the data in a time sequential manner, e.g., when some object flies to the user, the auxiliary detects the flying object by use of the fact that the contour increases sequentially in time. Then, the control signal forming operation part 14b of the image processing unit 14 forms a control signal for vibrating the actuator array 16 in a region corresponding to the flying object, and the actuator control unit 15 (15a, 15b) drives the actuator array 16 and vibrates the pins 26 based on the control signal. The frequency of vibration is set to, e.g., a higher frequency than the frequency for the above hole to increase the emergency.

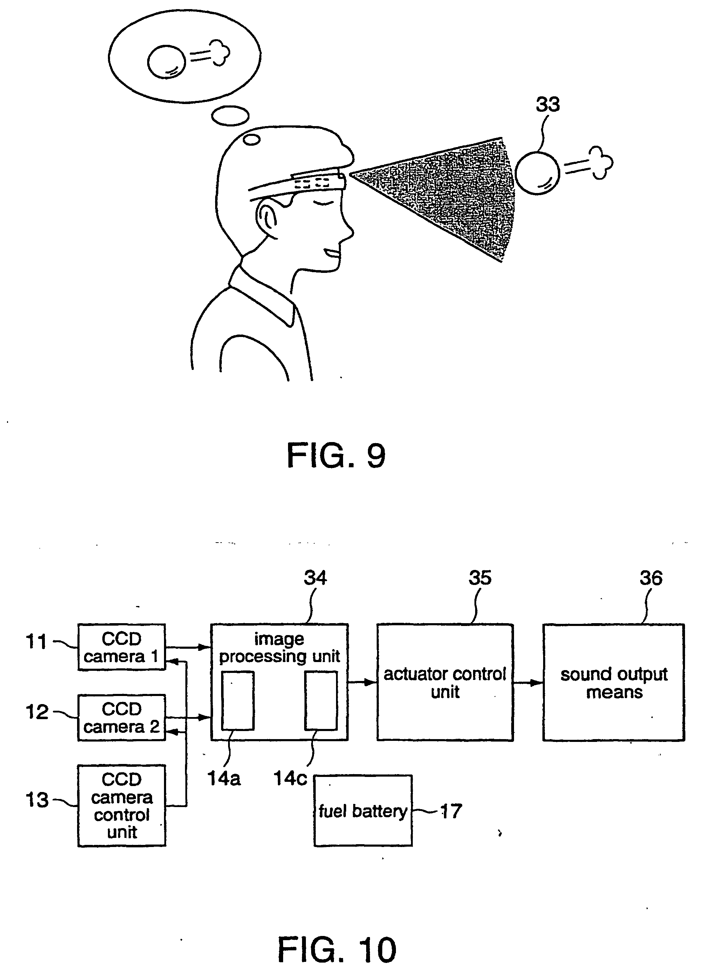

Fig. 9 is a diagram showing an example of a case where a ball 33 is flying. The control signal forming operation part 14b of the image processing unit 14 detects the ball 33 (flying object) and forms a control signal for vibrating the actuator array 16 of a region corresponding to the ball, and the actuator control unit 15 (15a, 15b) drives the actuator array 16 and vibrates the pins 26 based on the control signal. Thereby the user can identify the fact that something is flying at himself.

How to cope with an obstacle is different in each state whether the user is walking or standing still. When the user is standing still, for example, the control signal forming operation part 14b of the image processing unit 14 can correspond to a case of pressing danger by detecting objects of a predetermined area at a distance of 5 m or more and objects in motion, recognizing a state of relatively distant (e.g. 5 to 50m) surroundings (identifying what state of place he is in) and detecting the objects in motion. Moreover, in finding the contour of a picked-up object, the control signal forming operation part 14b of the image processing unit 14 stores the data in a time sequential manner and determines whether the user is walking or stopping based on whether the contour gets larger or not. Furthermore, when the control signal forming operation part 14b of the image processing unit 14 detects that the user is walking or detects a flying object, although in both cases the contour of the picked-up objects enlarges, it can discriminate between them, because the entire contour enlarges in the former case and a part of contour enlarges in a short time in the latter case.

Above, examples wherein the user is informed about the existence of an obstacle by driving the actuator array 16 were illustrated, but the user may also be informed about the existence of an obstacle aurally, i.e., by sound.

Fig. 10 is a block diagram showing the circuit construction of an auxiliary 20. It comprises the two CCD cameras 11, 12, the CCD camera control unit 13, an image processing unit 34, a sound signal forming unit 35, a sound output means (e.g., an earphone) 36 and the battery 17. The two CCD cameras 11, 12 are controlled by the CCD camera control unit 13, pick up images at different angles, respectively, and output the image pickup signals to the image processing unit 34. The image processing unit 34 is composed of a distance-measuring operation part 14a and a stereo shape discriminating operation part 14c. Similarly to the above case, the distance-measuring operation part 14a inputs the image signals from the CCD cameras 11, 12 for image processing, measures the distance and forms the stereo image information (three-dimensional information). The stereo shape discriminating operation part 14c contrasts the stereo image information with pre-stored stereo image information and determines what kind of information the stereo image information is. For example, it is known that an obstacle is a tree and it is also known how many meters this tree is located ahead of the user, therefore this information is output to the sound signal forming unit 35. The sound signal forming unit 35 forms a sound signal based on the information and generates a sound, "There is a tree 3 m ahead to the right", from the sound output means 36 to inform the user about the existence of the obstacle.

This example, which is useful in case the moving path of the user is previously known, prestores stereo image information (three-dimensional information) about a moving path and the surrounding obstacles and can concretely specify the obstacles to give guidance to the user by contrasting the stereo image information with the stereo image information (a three-dimensional information) formed by the image signals from the CCD cameras 11, 12. Moreover, even if this example cannot concretely specify the types of obstacle, it can inform the user about the existence of the obstacles.

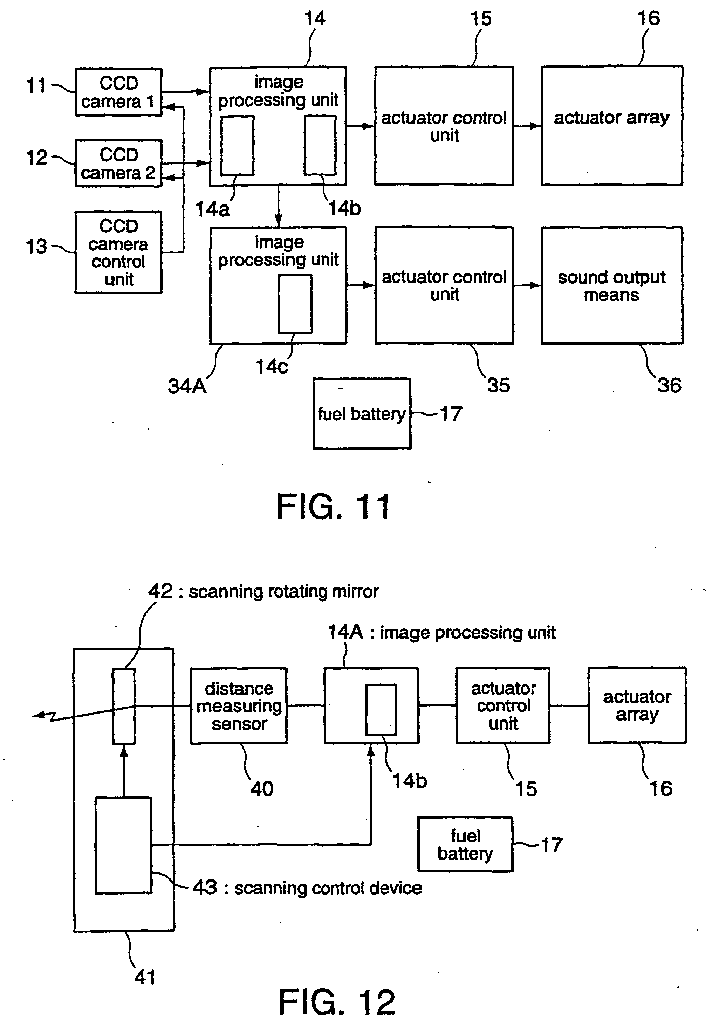

Fig. 11 is a block diagram showing the circuit construction of an auxiliary 20, which comprises the two CCD cameras 11, 12, the CCD camera control unit 13, the image processing unit 14, the actuator control unit 15, the actuator array 16, and the battery 17, and the image processing unit 34A, the sound signal forming unit 35, and the sound output means (e.g., an earphone) 36. Hence, it combines the above auxiliary of Fig. 1 and the above auxiliary of Fig. 10.

In this auxiliary 20, the two CCD cameras 11, 12 are controlled by the CCD camera control unit 13, to pick up images at different angles, respectively, and output their image pickup signals to the image processing unit 14. The image processing unit 14 receives the image signals from the CCD cameras 11, 12 for image processing, forms stereo image information (three-dimensional information), further converts the stereo image information to two-dimensional information to form a control signal for controlling the actuator array 16 and outputs it to the actuator control unit 15. The actuator control unit 15 drives the actuator array 16 and informs the user about surrounding conditions picked up by the two CCD cameras 11, 12. The image processing unit 34A (the stereo shape discriminating operation part 14c) receives the stereo image information (three-dimensional information) from the image processing unit 14, then contrasts the stereo image information with pre-stored stereo image information to determine its type. Similarly to the above case, for example, if it is known that an obstacle is a tree and it is also known how many meters this tree is located ahead of the user, corresponding information is output to the sound signal forming unit 35. The sound signal forming unit 35 forms a sound signal and generates a sound, "There is a tree 3 m ahead to the right", from the sound output means 36 to inform the user about the existence of the obstacle.

This example transmits more reliable information because it informs the user about the existence of obstacles through both the actuator array 16 and the sound output means 36. Moreover, the previous examples are also similarly applicable to this example.

Examples wherein the measurement of distance to an obstacle was made by using two CCD cameras 11, 12 were illustrated above, but a distance sensor may be used in place of the two CCD cameras 11, 12. In this case, the distance sensor is scanned to pick up images of a predetermined region ahead of the user. After the distance to the obstacle is obtained, processing is same as above.

Fig. 12 is a block diagram showing the circuit construction of an auxiliary 20 in which a distance sensor 40 and a scanning mechanism 41 for scanning the distance sensor 40 are provided in place of the two CCD cameras 11, 12. The scanning mechanism 41 is composed of a scanning rotating mirror 42 and a scanning control device 43. The scanning control device 43 measures the distance to the obstacle ahead of the user by controlling the scanning rotating mirror 42 to scan the measured sites of the distance sensor 40. Similarly to above, an image processing unit 14A (control signal forming operation part 14b) forms a control signal and outputs it to an actuator control unit 15 to drive an actuator array 16 based on the distance to the obstacle (three-dimensional information). This example may also be combined with the one of Fig. 10.

Battery 17 shown as power source for the auxiliary may be any means capable of providing electric power such as a fuel battery, a dry battery, a secondary battery, etc.. Furthermore, the examples mounted with various tools to a headband were illustrated, but they may also be mounted to hat or clothes and so on.

## Claims

1. A walking auxiliary for a person with dysopia, comprising:

   a distance-measuring means (11, 12, 14a) adapted to measure the distance to obstacles, operational means (14) adapted to form three-dimensional information from the measured distances and to output a control signal, multiple actuators (18), and a controlling means (15) adapted to drive the actuators (18) and to transmit to the user the existence of an obstacle somatosensorially based on the control signal,

   **characterized in that** the operational means (14) is adapted to convert the three-dimensional information to two-dimensional information and to output the two-dimensional information as said control signal, to detect whether the user is in a state of walking based on a change of the distance to the obstacle and to vary the formed two-dimensional information according to the state, and also to detect an obstacle within a predetermined distance and to form two-dimensional information of the obstacle in case the user is in the state of walking.

2. The walking auxiliary according to Claim 1, **characterized by** the operational means (14) adapted to add specific information to the two-dimensional information of adjacent obstacles among obstacles within the predetermined distance and to drive the actuators (18).

3. The walking auxiliary according to Claim 1 or 2, **characterized by** multiple actuators (18) being disposed in a matrix.

4. The walking auxiliary according to any of Claims 1 to 3, **characterized by** a sound signal forming means (35) adapted to form and output a sound signal based on the three-dimensional information and a sound output means (36) adapted to convert the sound signal to a sound and to output it.

5. The walking auxiliary according to Claim 4, **characterized by** the sound signal forming means (35) adapted to form and output a sound signal based on the three-dimensional information of an obstacle within a predetermined distance.

6. The walking auxiliary according to Claim 4 or 5 **characterized by** the sound signal forming means (35) adapted to compare the three-dimensional information and pre-registered three-dimensional information of the obstacle and, if both are consistent, to form a sound signal corresponding to information specifying the obstacle.

7. The walking auxiliary according to any of Claims 1 to 6, **characterized by** the distance-measuring means comprising a distance sensor and a scanning means adapted to scan the distance sensor.

8. The walking auxiliary according to any of Claims 1 to 6, **characterized by** the distance-measuring means being provided with plural image pickup means (11, 12) disposed in different positions and a distance-measuring operation part (14a) adapted to process the image pickup signals from the image pickup means and to find the distance to obstacles.

9. The walking auxiliary according to any of Claims 1 to 8, **characterized by** either the means and/or the actuators (18) being mounted to a headband (21).

## Patentansprüche

1. Gehhilfsmittel für eine sehbehinderte Person umfassend:

   eine Abstandsmessvorrichtung (11, 12, 14a), dazu ausgebildet, den Abstand zu Gegenständen zu messen, Betriebsmittel (14), die ausgebildet sind, dreidimensionale Informationen von den gemessenen Abständen zu bilden und ein Steuersignal auszugeben, mehrere Aktuatoren (18), und Steuermittel (15), die ausgebildet sind, die Aktuatoren (18) anzutreiben und die Existenz eines Gegenstandes somatosensorisch basierend auf dem Steuersignal an den Benutzer zu übertragen,

   **dadurch gekennzeichnet, dass** die Betriebsmittel (14) dazu ausgebildet sind, die dreidimensionalen Informationen in zweidimensionale Informationen umzuwandeln und die zweidimensionalen Informationen als das Steuersignal auszugeben,

festzustellen, auf der Basis einer Änderung des Abstands zum Gegenstand, ob der Benutzer in einem Zustand des Gehens ist und die gebildeten zweidimensionalen Informationen gemäß dem Zustand zu variieren, und

außerdem einen Gegenstand innerhalb eines vorbestimmten Abstandes zu ermitteln, und zweidimensionale Informationen des Gegenstandes zu bilden, falls der Benutzer sich in einem Zustand des Gehens befindet.

2. Gehhilfsmittel gemäß Anspruch 1, **dadurch gekennzeichnet, dass** die Steuermittel (14) dazu ausgebildet sind, spezielle Informationen zu den zweidimensionalen Informationen von benachbarten Gegenständen unter Gegenständen innerhalb des vorbestimmten Abstandes hinzuzufügen und die Aktuatoren (18) anzutreiben.

3. Gehhilfsmittel gemäß Anspruch 1 oder 2, **dadurch gekennzeichnet, dass** mehrere Aktuatoren (18) in einer Matrix angeordnet sind.

4. Gehhilfsmittel gemäß einem der Ansprüche 1 bis 3, **gekennzeichnet durch** eine Vorrichtung zur Erzeugung eines Tonsignals (35), die dazu ausgebildet ist, ein akustisches Signal basierend auf den dreidimensionalen Informationen zu erzeugen und auszugeben und eine Tonausgabevorrichtung (36), die dazu ausgebildet ist, ein Tonsignal in einen Ton umzuwandeln und ihn auszugeben.

5. Gehhilfsmittel gemäß Anspruch 4, **dadurch gekennzeichnet, dass** die Vorrichtung zur Erzeugung eines Tonsignals (35) dazu ausgebildet ist, ein Tonsignal basierend auf den dreidimensionalen Informationen eines Gegenstandes innerhalb eines vorbestimmten Abstandes zu erzeugen und auszugeben.

6. Gehhilfsmittel gemäß Anspruch 4 oder 5, **dadurch gekennzeichnet, dass** die Vorrichtung zu Erzeugung eines Tonsignals (35) dazu ausgebildet ist, die dreidimensionalen Informationen mit vorregistrierten dreidimensionaler Informationen des Gegenstandes zu vergleichen und, wenn beide übereinstimmen, ein Tonsignal entsprechend einer den Gegenstand spezifizierenden Information zu erzeugen.

7. Gehhilfsmittel gemäß einem der Ansprüche 1 bis 6, **dadurch gekennzeichnet, dass** die Abstandsmessvorrichtung einen Abstandssensor und ein Abtastmittel umfasst, die dazu ausgebildet sind, den Abstandssensor abzutasten.

8. Gehhilfsmittel gemäß einem der Ansprüche 1 bis 6, **dadurch gekennzeichnet, dass** die Abstandsmessvorrichtung mit mehreren Bildaufnahmemitteln (11, 12) versehen ist, die in verschiedenen Positionen angeordnet sind und einen Abstandsmessbetriebsteil (14a), das dazu ausgebildet ist, die Bildaufnahmesignale von den Bildaufnahmemitteln zu verarbeiten und die Entfernung zu den Gegenständen zu ermitteln.

9. Gehhilfsmittel gemäß einem der Ansprüche 1 bis 8, **dadurch gekennzeichnet, dass** die Mittel und/oder die Aktuatoren (18) an einem Kopfband (21) montiert sind.

## Revendications

1. Aide à la marche pour personne atteinte de dysopie, comprenant :

un moyen (11, 12, 14a) de mesure de distance conçu pour mesurer la distance par rapport à des obstacles,
un moyen (14) opérationnel conçu pour former des informations en trois dimensions à partir des distances mesurées et pour émettre un signal de commande,
de multiples actionneurs (18), et
un moyen (15) de commande conçu pour commander les actionneurs (18) et pour informer l'utilisateur de la présence d'un obstacle de manière somatosensorielle sur la base du signal de commande,

**caractérisée en ce que** le moyen (14) opérationnel est conçu

pour convertir les informations en trois dimensions en informations en deux dimensions et pour transmettre les informations en deux dimensions sous la forme du signal de commande,
pour détecter si l'utilisateur est dans un état de marche sur la base d'un changement de la distance par rapport à l'obstacle et pour modifier les informations en deux dimensions en fonction de l'état, et
pour détecter également un obstacle sur une distance prédéterminée et pour former les informations en deux dimensions correspondant à l'obstacle si l'utilisateur est dans l'état de marche.

2. Aide à la marche selon la revendication 1, **caractérisée en ce que** le moyen (14) opérationnel est conçu pour ajouter des informations spécifiques aux informations en deux dimensions correspondant aux obstacles voisins parmi les obstacles sur une distance prédéterminée et pour commander les actionneurs (18).

**3.** Aide à la marche selon la revendication 1 ou 2, **caractérisée en ce que** les multiples actionneurs (18) sont disposés en une matrice.

**4.** Aide à la marche selon l'une quelconque des revendications 1 à 3, **caractérisée par** un moyen (35) formant un signal de son conçu pour former et émettre un signal de son basé sur les informations en trois dimensions et un moyen (36) de sortie de son conçu pour convertir le signal de son en un son et pour l'émettre.

**5.** Aide à la marche selon la revendication 4, **caractérisée en ce que** le moyen (35) formant un signal de son est conçu pour former et émettre un signal de son basé sur les informations en trois dimensions correspondant à un obstacle sur une distance prédéterminée.

**6.** Aide à la marche selon la revendication 4 ou 5, **caractérisée en ce que** le moyen (35) formant un signal de son est conçu pour comparer les informations en trois dimensions et des informations en trois dimensions préenregistrées correspondant à l'obstacle et, si les informations correspondent, pour former un signal de son correspondant aux informations spécifiant l'obstacle.

**7.** Aide à la marche selon l'une quelconque des revendications 1 à 6, **caractérisée en ce que** le moyen de mesure de distance comprend un capteur de distance et un moyen de balayage conçu pour analyser le capteur de distance.

**8.** Aide à la marche selon l'une quelconque des revendications 1 à 6, **caractérisée en ce que** le moyen de mesure de distance est muni d'une pluralité de moyens (11, 12) analyseur d'images disposés dans différentes positions et une partie (14a) de commande de mesure de distance conçue pour traiter les signaux d'analyse d'images provenant des moyens analyseur d'images et pour déterminer la distance par rapport aux obstacles.

**9.** Aide à la marche selon l'une quelconque des revendications 1 à 8, **caractérisée en ce que** les moyens et/ou les actionneurs (18) sont montés sur un serre-tête (21).

FIG. 1

FIG. 2

FIG. 3

# FIG. 4

```
                    ┌─────────────────┐
                    │      Start      │
                    └─────────────────┘
                             │
         ┌──────────────────▶│
         │          ┌─────────────────┐
         │          │ Incorporation of│
         │          │   image data    │╮  S1
         │          └─────────────────┘
         │                   │
         │          ┌─────────────────┐
         │          │Distance calculation│╮  S2
         │          └─────────────────┘
         │                   │
         │   (preparation of three-dimensional information)
         │          ┌─────────────────┐
         │          │  Conversion to  │
         │          │ plane information│╮  S3
         │          └─────────────────┘
         │                   │
         │   (conversion to two-dimensional information)
         │          ┌─────────────────┐
         │          │Formation and output│
         │          │ of control signal│╮  S4
         │          └─────────────────┘
         │                   │            S5
         │          ╱─────────────────╲
         └─────────〈 Power source OFF  〉
                    ╲─────────────────╱
                             │
                    ┌─────────────────┐
                    │      Stop       │
                    └─────────────────┘
```

# FIG. 5

11

FIG. 6

EP 1 293 184 B1

FIG. 7

FIG. 8

# FIG. 9

# FIG. 10

14   15   16

11 — CCD camera 1

14 — image processing unit

12 — CCD camera 2

13 — CCD camera control unit

14a   14b

actuator control unit — 15

actuator array — 16

image processing unit

34A   14c

actuator control unit — 35

sound output means — 36

fuel battery — 17

## FIG. 11

42 : scanning rotating mirror

14A : image processing unit

distance measuring sensor — 40

14b

actuator control unit — 15

actuator array — 16

43 : scanning control device

fuel battery — 17

41

## FIG. 12